# EUROPEAN PATENT SPECIFICATION

(11) **EP 0 401 421 B1**
(45) Date of publication and mention of the grant of the patent: **03.01.1996**
(21) Application number: 89111281.5
(22) Date of filing: 21.06.1989
(51) Int. Cl.: C12N 7/06

(54) **Non-reverting RNA viruses**
Nicht-zurückmutierende RNA-Viren
Des virus d'ARN non réversibles

(30) Priority: 08.06.1989 US 363611
(43) Date of publication of application: 12.12.1990
(73) Proprietor: AMERICAN CYANAMID COMPANY, Wayne, NJ 07470-8426 (US)
(72) Inventor: Weeks-Levy, Carolyn Louise, Thornwood, New York 10594 (US); Mento, Steven Joseph, Chester, New York 10918 (US)
(74) Representative: Wächtershäuser, Günter, Prof. Dr.

(56) References cited:
- EP-A- 0 325 768
- WO-A-89/06277
- BIOLOGICAL ABSTRACTS, vol. 84, 1987, page AB-369, abstract no. 107609; N. LA MONICA et al.: "A mouse model for poliovirus neurovirulence identifies mutations that attenuate the virus for humans"
- VIROLOGY, vol. 166, 1988, pp. 394-404, Academic Press, Inc.; Y.V. SVITKIN et al.: "Point mutations modify the response of poliovirus RNA to a translation initiation factor a comparison of neurovirulent and attenuated strains"
- NATURE, vol. 314, 11th April 1985, pp. 548-550; D.M.A. EVANS et al.: "Increased neurovirulence associated with a single nucleotide change in a noncoding region of the Sabin type 3 poliovaccine genome"
- J. GEN. VIROL., vol. 69, 1988, pages 1091-1096, SGM; P.D. MINOR et al.: "The effect of sequences in the 5' non-coding region on the replication of polioviruses in the human gut"
- CHEMICAL ABSTRACTS, vol. 104, 1986, page 173, abstract no. 83080r, Columbus, Ohio, US

## Description

This invention relates to a method for producing modified, highly attenuated polio virus strains having a low rate of replication and reversion. More particularly this invention relates to vaccines and the use of such vaccines comprising those attenuated polio viral strains.

### BACKGROUND OF THE INVENTION

Various positive strand RNA. viruses or picornaviruses are well known. Typical picornaviruses include polioviruses, echoviruses, coxsackieviruses and rhinovirus (common cold viruses).

Vaccines, to protect against diseases induced by such viruses, are prepared using live or killed viral varients or mutants that are attenuated. Attenuated viruses exhibit a lowered pathogenicity. For example, Albert Sabin in the 1950's isolated attenuated strains of the poliovirus by passing the virus in tissues from different animal hosts in cultured cells, until varients were selected having lost their capacity to cause disease [A. J. Sabin et al., "History Of Sabin Attenuated Poliovirus Oral Live Vaccine Strains", J. Biol. Stard., 1, p. 115 (1973)]. Virus-based vaccines are useful because they *are* able to replicate and induce adequate immunity through the production of antibodies. However, live vaccines have the additional benefit of inducing immunity at the site of infection whereas killed vaccines are limited to inducing serum immunity. Live virus vaccines are commonly distributed because of the ease of administration, low cost and the rapidity vaccine recipients develop long-lasting immunity. Unfortunately, the live viruses may revert, i.e., from the live viral pool may emerge new varients that are capable of replicating to cause disease, either once in the vaccine recipient or during manufacture. It would therefore be of interest to produce a strain that is less likely to revert for use as a vaccine.

### SUMMARY OF THE INVENTION

The present invention solves the problem referred to above by a method for providing highly attentuated polio virus strains that have a lowered potential of reverting to virulence as defined in claims 1 and 2. Polio viral strains of this invention are useful in vaccines to protect recipients or those in close contact from viral induced disease.

One embodiment of a process of this invention for producing these attentuated polio viral strains is the modification of a polio viral genome to decrease the base pairing of the viral genome to itself.

A second embodiment of a process of this invention is the modification of a polio viral genome in order to decrease base pairing between the viral RNA and mammalian host ribosomal RNA. The process of this invention modifies viral RNA bases
in regions containing complementarity with host ribosomal RNA by decreasing base pairing.

Such modifications of base pairing comprise the steps of isolating polio viruses, reverse transcribing the RNA to give cDNA copies; mutagenizing the cDNA to cause the effects referred to above in any of the two embodiments; transfecting cells capable of producing viral RNA with the mutagenized polio viral cDNA; and, culturing to produce attenuated polio virus. These modifications are effective to reduce the transcriptional or translational efficiency of polio viral strains.

An object of this invention is to employ, the attenuated polio viruses in a live vaccine. Administration of the vaccine may be via any of the conventional accepted modes of administering live vaccines.

### BRIEF DESCRIPTION OF THE DRAWINGS

Figure 1 depicts a loop predicted by matching-up complementary bases from the published cDNA polio genome between bases 370 and 471 of the 5' noncoding region [Toyoda et al., J. Mol. Biol., 174, p. 561 (1984)], and the resulting modified loop after mutagenesis.

Figure 2 is a schematic depiction of the 7500 b.p. poliovirus genome having complementarity with cellular mouse ribosomal RNA [McClure and Perrault, Nucleic Acids Res., 13, pp. 6797-6816 (1985)].

Figure 3 depicts the interaction of published 28S rat ribosomal sequences [Genbank, "NucleotideSequences", Vol. 1, p. 243, Oxford Press (1985)] with polio cDNA sequences (Toyoda et al., supra).

Figure 4 depicts conserved DNA sequence regions A and B in the RNA sequence of attentuated strain Sabin 3.

Figure 5 depicts two site-directed mutations in the RNA sequence of attenuated strain Sabin 1.

Figure 6 depicts four site-directed mutations in the RNA sequence of attenuated strain Sabin 2.

Figure 7 depicts three site-directed mutations in the RNA sequence of attenuated strain Sabin 3.

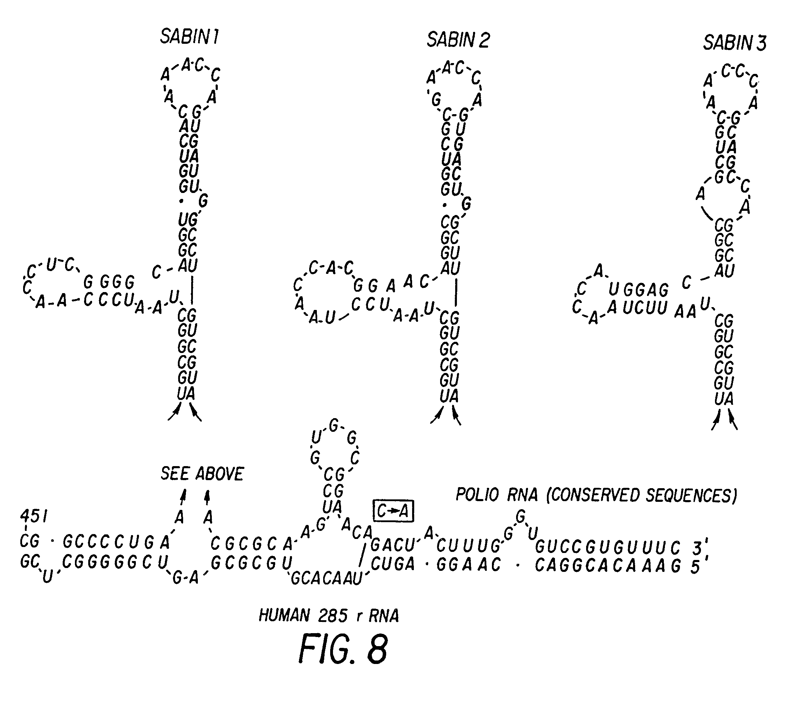

Figure 8 depicts a site-directed mutation in the RNA sequence of attenuated Sabin strains 1, 2, and 3 in areas not having complementarity with human 28S rRNA.

### DETAILED DESCRIPTION OF THE INVENTION

The present invention relates to a process for the production of highly attenuated polio viral strains. Attenuated polio viral strains, as defined by this invention, have a lowered transcriptional or translational efficiency which results in slower replication of viruses. Because the generation of new viral populations is reduced, there is a less likelihood of reversion which produces a viral variant that causes disease.

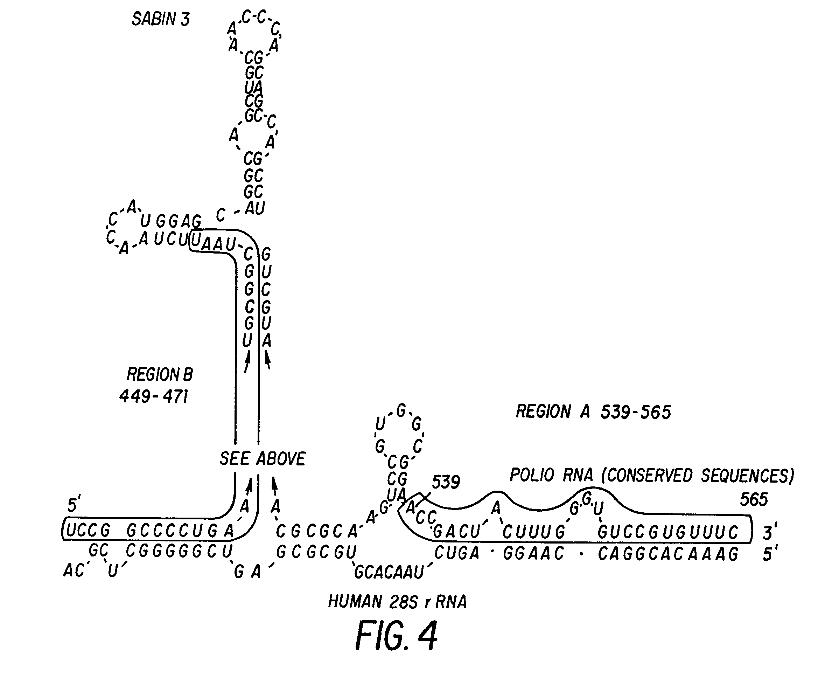

As described above, in the first embodiment of this invention, the polio viral RNA genome has been altered by decreasing the base pairing to itself. The polio viral RNA that is modified, is located in the looped regions of the genome. Specifically, this mutagenesis is carried out with polio virus cDNA in the region corresponding to base sequences 486-516 of Figure 4. In these loops, caused by base-base interactions, there are unbound and bound base residues. We choose to substitute according to the method described below, unbound bases with bases having complementarity with another unbound base. While not wishing to be bound by theory, we believe an increase in base pairing stabilizes viral RNA folding, which in turn makes it more difficult to unwind for initiation of transcription or translation. We also believe modified base pairing involves changes the secondary structure of polio viral RNA, which disrupts its binding to host ribosomal RNA during transcription or translation or which prevents reversion. A decrease in base pairing can also change the secondary structure resulting in the disruption of translational or transcriptional efficiency. These disruptions reduce the efficiency of transcription or translation, hence viral production decreases. Accordingly, viral strains modified by the processes of this invention are characterized by a reduced rate of replication and reversion as compared with the native or other unmodified strains. A more attenuated strain results because the rate of producing any potential viral varient that may induce disease is lowered.

Also, as described above as a second embodiment of this invention, the polio viral genome is modified in regions containing complementarity with host ribosomal RNA to provide highly attenuated viruses. Modifications of this sort includes decreasing the base pairing in regions not containing complementarity with host ribosomal RNA. Specifically, this mutagenesis is carried out with polio virus cDNA such that the mutagenesis decreases base pairing in the regions corresponding to base sequences 449-460, 542-545, 547-551 and 555-565 in Figure 4.

We believe these alterations also disrupt the efficiency of translation, which leads to lower production of any potential viral variant.

The invention also relates to a process for producing highly attenuated polio viruses of this invention. In the preferred embodiment of this process we purify viruses in order to remove any cellular debris, followed by synthesizing full length viral double stranded cDNA according to the method of Racaniello et al. [V. R. Racaniello et al., "Molecular Cloning Of Poliovirus cDNA And Determination Of The Complete Nucleotide Sequence of The Viral Genome", Proc. Natl. Acad. Sci. U.S.A., 78, p. 4887 (1981)]. Next, according to the first embodiment of this invention, polio viral cDNA that correspond to the RNA bases in the polio viral genome looped structure are mutagenized using conventional techniques to
decrease base pairing. Also, according to the second embodiment of this invention, the polio viral cDNA corresponding to regions of the polio viral RNA containing complementarity with host ribosomal RNA is mutagenized to decrease the base pairing between the RNA genome and the ribosomal RNA.

After mutagenesis, cells capable of RNA production, for example CV-1, VERO, or Eela cells, are transfected with cDNA, using conventional techniques. Live virus is next detected, using known methods and sequenced by known techniques to determine if the RNA viral sequence corresponds to the mutagenized cDNA sequence. Finally, the degree of attenuation is detected. Our preferred method produces a chimeric cDNA containing the mutagenized polio cDNA (Sabin 1, 2, or 3) and sequences necessary for infectivity in mice [Murray et al., "Polio Virus Host Range Is Determined By A Short Amino Acid sequence In Neutralization Antigenic Site-I," Science, 241, pp. 213-15 (1988)]. Such viruses made from this chimeric plasmid are used to perform a mouse neurovirulence test. Another possible method uses an assay which measures the rate of production of viral protein labelled with 35S-methionine.

The polio viral strains of this invention can be employed in a vaccine using known methods to produce live vaccines. The resulting vaccine will contain an amount of live virus effective in the recipient to protect against viral infection. Administration of those vaccines may be via any of the conventional accepted modes of administration of live vaccines. In order that this invention may be better understood, the following example is set forth. This example is for the purposes of illustration only and is not to be construed as limiting the scope of the invention.

### EXAMPLE

### 1. Virus Purification

We used polioviruses (available from the F.D.A.) maintained in culture conditions described by A. Sabin et al., "Studies On Varients Of Poliomyelitis Virus", J. Exp. Med., 99:551 (1954). Specifically we infected primary monkey kidney tissue at a low multiplicity of infection and incubated the virus at 34° at pH 7.3. To remove any cellular debris we centrifuged harvest fluid containing the strain at low speed (2000 RPM for 20 minutes). We precipitated the virus by adding NaCl (.12M) and polyethylene glycol 8000 (12% by weight) followed by stirring overnight at 4°C. The precipitated virus was collected by centrifugation at 10,000 RPM for 20 minutes.

After resuspending the virus pellet in a total volume of 1.2 ml of TNE (10 mM Tris-HCl, 100 mM NaCl, 1 mM EDTA, at pH 8.0), we added NONIDET P-40 until the final concentration was 1% by weight. We next layed the virus onto 15%-45% sucrose gradients [P. P. Minor, "Comparative Biochemical Studies Of Type 3 Polio Virus", J. Virol, 34, p. 73 (1980)], and spun them at 12,300 RPM for 16 hours in a SW 28.1 rotor. We collected 1 ml. fractions from the top of the gradient. Twenty-five microliter samples were taken from each sample and were run on discontinuous 15% polyacrylamide gels. We located the poliovirus in the fractions by visualization of the capsid proteins in the gel by silver straining the gels [W. Wray et al., "Silver Staining Of Proteins In Polyacrylamide Gels", Analy. Bioch., 118, p. 197 (1981)]. Sucrose fractions containing poliovirus were pooled and sodium dodecyl sulfate (SDS) was added to a final concentration of 0.5%. We next extracted virus from the pooled sucrose fractions with an equal volume of phenol containing chloroform and isoamyl alcohol (25:24:1) once, and back extracted the phenol once. Aqueous layers were pooled and 0.5 volumes of 7.5M ammonium acetate was added. Using 2.5 volumes of absolute ethanol and freezing at -70°C overnight poliovirus RNA was precipitated. Afterwards, we resuspended the RNA pellet in water for injection (WF1) that had been previously treated with diethyl pyrocarbonate (DEP) and reprecipitated the RNA by adding 2.5 volumes of absolute ethanol. After repelleting the RNA and washing twice with ice cold 70% ethanol, the final RNA pellet was resuspended in 20 ul of DEP treated WFI. We next sequence the RNA according to the method of Sanger [Sanger et al., "DNA Sequencing With Chain Terminating Inhibitors", Proc. Nat'l. Acad. Sci. USA, 74, p. 5463 (1973)] with modifications for RNA and problems with secondary structure associated with RNA templates [D. C. DeBorde et al., "Resolution Of A Common RNA Sequencing Ambiguity By Terminal Deoxynucleotidyl Transferase", Analy. Bioch., 157, p. 275 (1986)]. For example, terminal deoxynucleotidyl transferase is used to eliminate artifacts due to secondary structure.

### 2. Synthesis of Full Length Poliovirus Double-Stranded cDNA

RNA from the previous step is used as a template for the synthesis of double stranded cDNA Racaniello et al., supra. Using oligo(dg).oligo(dc) tailing method of Bothwell et al. [A. L. M. Bothwell, Cell, 24, p. 625 (1981)], double stranded cDNA are, inserted into the PstI site of plasmid pBR322. Tetracycline-resistant clones are screened for inserts by colony hybridization, using poliovirus cDNA probe [M. Grunstein et al., Proc. Nat'l. Acad. Sci USA, 72, p. 3961 (1975)]. Using nucleotide sequence analysis and restriction enzyme mapping techniques the inserts are aligned on the viral genome (V. R. Racanicello et al., infra). Finally, partial poliovirus cDNA clones are ligated into a single, full length clone, at their common cleavage sites [Racanicello et al., "Cloned Poliovirus cDNA Is Infectious In Mammalian Cells", Science, 214, p. 916 (1981)].

### 3. Mutagenesis of Poliovirus cDNA

In accordance with this invention, poliovirus cDNA from the previous step corresponding to unbound bases and a stem and loop structure of the viral genome are mutagenized to decrease base pairing with other unbound bases located within the loop or outside of the loop in the linear portion of the genome.

As a means for mutagenizing unbound base pairs, site-directed mutagenesis of poliovirus cDNA is employed using the M13 method [C. A. Hutchinson III et al., "Mutagenesis At A Specific Position In A DNA Sequence", J. Biol. Chem., 253, p. 655 (1978); A. Razin et al., "Efficient Correction of A Mutation By Use Of A Chemically Synthesized DNA", Proc. Natl. Acad. Sci. USA, 75, p. 4268 (1978)].

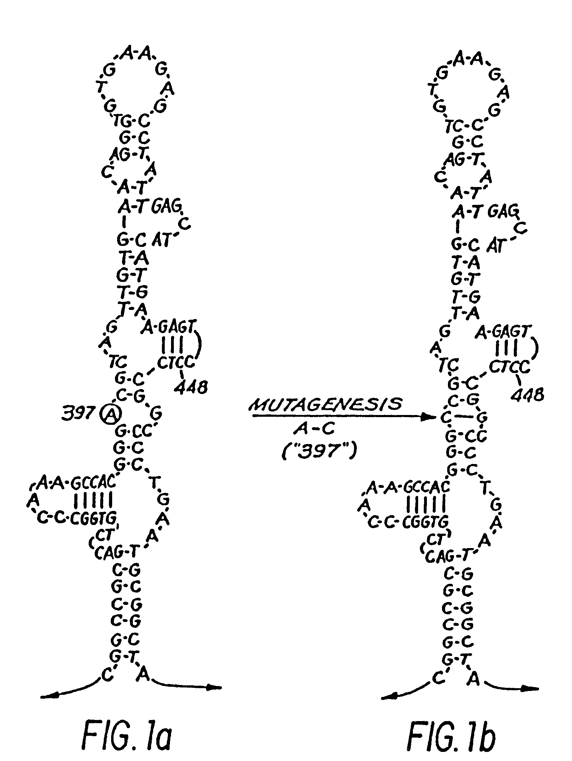

Fig. 1 depicts a cDNA stem and loop structure of the polio genome with unbound bases.

In accordance with the second embodiment of this invention, we mutagenize poliovirus cDNA corresponding to the regions containing complementarity with rat ribosomal RNA, by decreasing base pairing between the poliovirus genome and rat ribosomal RNA.

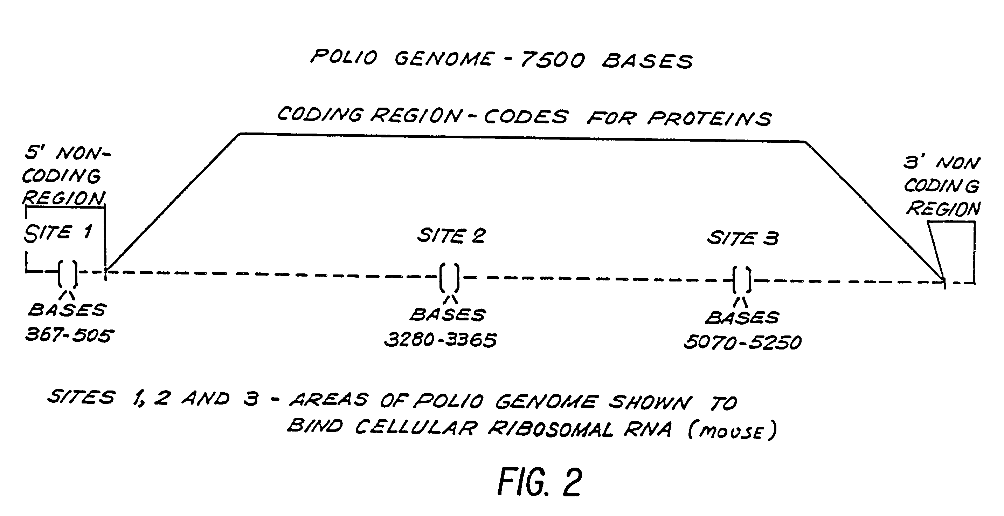

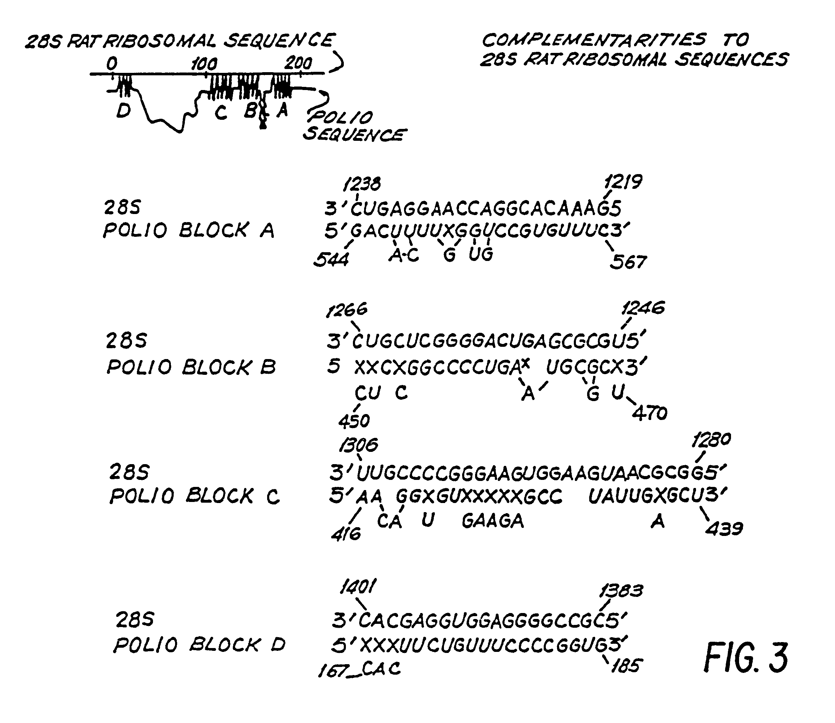

As shown in Figure 2, areas of the polio genome have been shown to bind with cellular mouse RNA. Within these areas are sequences that have complementarity with rat ribosomal RNA (see Fig. 3). Base residues within these sequences are mutagenized by the above method to either decrease base pairing with the rat ribosomal RNA.

Next, we determined which sequences correspond to the stem and loop structure in the viral RNA genome after interaction with human 28S rRNA. First, we aligned picornaviral 5' untranslated region sequences, including polio sequences and published coxsackie and rhinovirus sequences. This alignment was similar to that of Rivera et al., "Comparative Sequence Analysis of The 5' Noncoding Region Of The Enteroviruses And Rhinoviruses", Virology, 165, pp. 42-50 (1988). After observing four major blocks of conserved sequences we decided to focus on two blocks of conserved sequences, region A including bases 539-565 and region B including bases 449-471 of poliovirus attenuated strain 3 as depicted in Figure 4. Next, we searched for human 28S rRNA sequences complementary to the conserved regions A and B. We found five contiguous stretches of human 285 rRNA complementary to part of both of our regions A and B as shown in Table I.

**TABLE I**

| REGIONS OF 28S rRNA COMPLEMENTARY TO CONSERVED POLIOVIRAL REGIONS | | |
|---|---|---|
| | Complementary Sequence Range | |
| Region | A | B |
| 1 | 3454-3474 | 3477-3496 |
| 2 | 3940-3962 | 3967-3983 |
| 3 | 1301-1320 | 1329-1346 |
| 4 | 3171-3190 | 3191-3215 |
| 5 | 4052-4074 | 4075-4093 |

Polioviral base sequences between conserved regions A and B interacting with 28S rRNA form the stem and loop RNA structure in accordance with this invention. These sequences are mutagenized to decrease base pairing within the structure according to the invention.

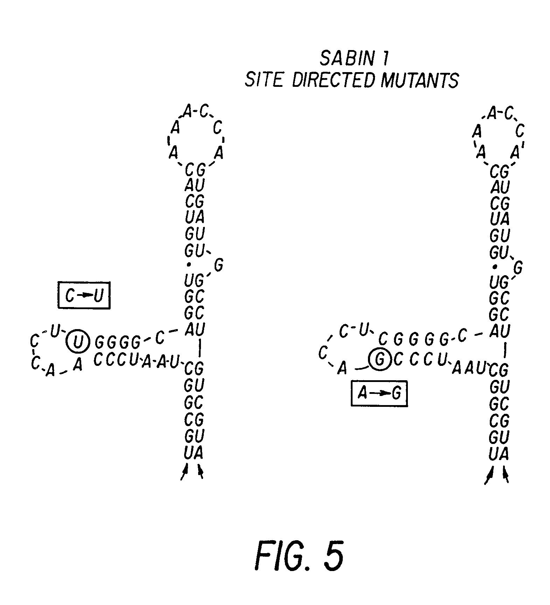

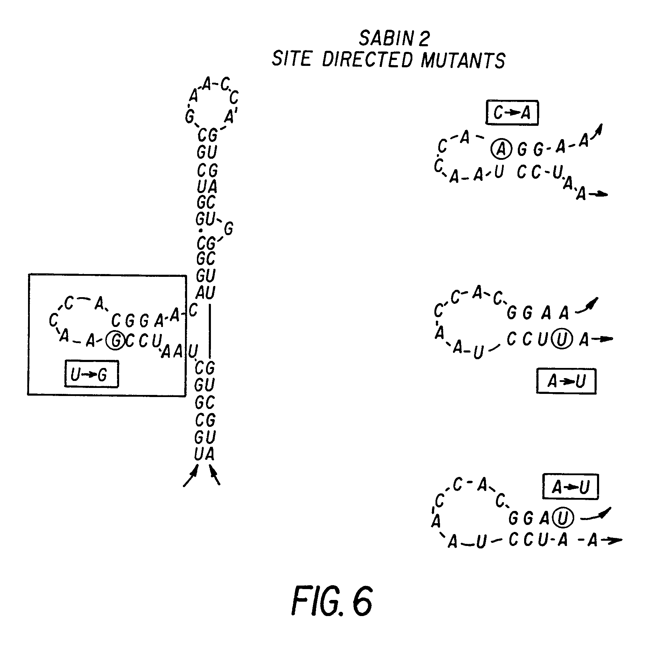

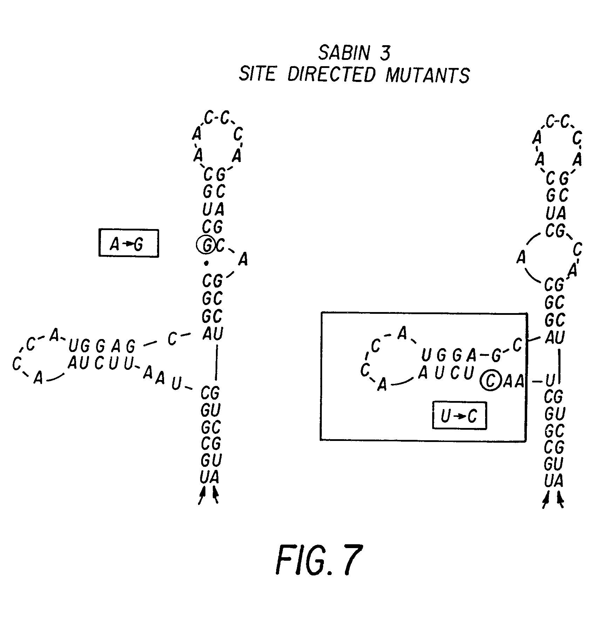

Referring now to Figures 5-7, we have depicted therein RNA stem and loop structures between conserved regions A and B having complementarity with 28S rRNA formed by the viral sequences of Sabin 1, 2 and 3, attenuated viruses. Various RNA base changes are indicated, in order to increase the RNA-RNA binding of unbound bases or increase the stability of bases already bound. Other unbound bases can be substituted in this manner in order to decrease base pairing.

Polioviral base sequences within conserved regions A and B having complementarity with 28S rRNA are mutagenized. Polioviral base sequences within these regions are mostly bound to 28S rRNA thereby forming the complementary structure. The number of bound bases is decreased in accordance with the invention.

### 4. Transfection of Cells With Mutagenized Poliovirus Clone

Having mutagenized poliovirus cDNA, host cells capable of RNA production, for example, VERO or HeLa cells, are transfected with plasmid cDNA. Transfection is conducted using a calcium-phosphate technique [B. A. Parker, J. Virol., 31, p. 360 (1979)], modified by treating the host cells with electroporation to help them take up this cDNA (apparatus from Promega, Madison, Wisconsin).

### 5. Detection Of Live Poliovirus

After transfection of the mutagenized polio cDNA, production of virus is detected by looking for the active production of poliovirus by cell lysis, also called cytopathic effect (CPE) [T. H. Donnebacker, "Correlation Of The Stage Of Cytopathic Change With The Release Of Poliomyelitis Virus", Virology, 2, p. 399 (1956)]. To confirm that the RNA viral sequence corresponds to the mutagenized cDNA sequence, the RNA genome is sequenced according to the method of Sanger et al., supra.

### 6. Determination Of The Degree Of Attenuation

The rate of production of viral protein is used as an assay for attenuation, comparing the currently used Sabin strain and the strain of this invention. Viral protein is labelled in the presence of ³⁵S methionine and the protein products are separated by SDS-polyacrylamide electrophoresis, followed by autoradiographing and scanning the gel to determine the different levels of viral proteins and the degree of inhibition of host protein synthesis.

Another method detects the degree of attentuation in a mouse neurovirulence test. Chimeric plasmids containing polio cDNA mutagenized according to any of the embodiments of this invention combined with cDNA sequences capable of invoking infectivity in mice [Murray et al., supra] are produced to detect the degree of attenuation in a mouse neurovirulence test. Confirmation of increased attentuation or decreased neurovirulance is determined by comparing existing vaccine strains to newly made strains, in accordance with this invention, in a monkey neurovirulence test (21 C.F.R. 600-799, 1987).

### 7. Preparation Of The Live Vaccine

After assaying for attenuation the polio viral particle selected now becomes the master seed from which the working seed is derived. For example the master seed is infected in Vero cells on microcarriers contained in a bioreactor, harvested and purified for use in a vaccine.

While we have hereinbefore described a number of embodiments of this invention, it is apparent that our basic constructions can be altered to provide other embodiments which utilize the processes and compositions of this invention.

## Claims

1. A method for producing modified highly attenuated poliovirus strains characterized by having a reduced rate of reversion in a mammalian host, said method comprising:
a. isolating poliovirus sequences from viral particles;
b. preparing poliovirus cDNA from said poliovirus sequences;
c. mutagenizing said poliovirus cDNA in the region corresponding to base sequences 468-516 of Figure 4 to decrease base pairing of the cDNA to itself, such that said mutagenesis reduces transcriptional and translation efficiency of poliovirus strains that are produced from said poliovirus cDNA;
d. transfecting cells with said mutagenized poliovirus cDNA;
e. culturing said host cells; and
f. extracting poliovirus strains from said cells.

2. A method for producing modified highly attenuated poliovirus strains characterized by having a reduced rate of reversion in a mammalian host, said method comprising:
a. isolating poliovirus sequences from viral particles;
b. preparing poliovirus cDNA from said poliovirus sequences;
c. mutagenizing said poliovirus cDNA to decrease base pairing of the cDNA to ribosomal RNA from said mammalian host, such that said mutagenesis decreases base pairing in the regions corresponding to base sequences 449-460, 542-545, 547-551 and 555-565 in Figure 4 and thereby reduces transcriptional and translation efficiency of poliovirus strains that are produced from said poliovirus cDNA;
d. transfecting cells with said mutagenized poliovirus cDNA;
e. culturing said host cells; and
f. extracting poliovirus strains from said cells.

## Patentansprüche

1. Verfahren zur Herstellung modifizierter, hoch abgeschwächter Poliovirus-Stämme, die dadurch gekennzeichnet sind, daß sie eine verringerte Reversionsrate in einem Säugerwirt aufweisen, wobei das Verfahren umfaßt:
a. Isolieren von Poliovirus-Sequenzen aus Virusteilchen ;
b. Herstellen von Poliovirus-cDNA aus den Poliovirus-Sequenzen;
c. Mutagenisieren der Poliovirus-cDNA in der Region, die den Basensequenzen 468-516 der Figur 4 entspricht, um die Basenpaarung der cDNA mit sich selbst zu verringern, so daß die Mutagenese die Transkriptions- und Translationseffizienz von Poliovirus-Stämmen verringert, die aus der Poliovirus-cDNA erzeugt werden;
d. Transfizieren von Zellen mit der mutagenisierten Poliovirus-cDNA;
e. Kultivieren der Wirtszellen; und
f. Extrahieren von Poliovirus-Stämmen aus den Zellen.

2. Verfahren zur Herstellung modifizierter, hoch abgeschwächter Poliovirus-Stämme, die dadurch gekennzeichnet sind, daß sie eine verringerte Reversionsrate in einem Säugerwirt aufweisen, wobei das Verfahren umfaßt:
a. Isolieren von Poliovirus-Sequenzen aus Virusteilchen;
b. Herstellen von Poliovirus-cDNA aus den Poliovirus-Sequenzen;
c. Mutagenisieren der Poliovirus-cDNA, um die Basenpaarung der cDNA mit ribosomaler RNA aus dem Säugerwirt zu verringern, so daß die Mutagenese die Basenpaarung in den Regionen verringert, die den Basensequenzen 449-460, 542-545, 547-551 und 555-565 in Figur 4 entsprechen, und dadurch die Transkriptions- und Translationseffizienz von Poliovirus-Stämmen verringert, die aus der Poliovirus-cDNA erzeugt werden;
d. Transfizieren von Zellen mit der mutagenisierten Poliovirus-cDNA;
e. Kultivieren der Wirtszellen; und
f. Extrahieren von Poliovirus-Stämmen aus den Zellen.

## Revendications

1. Procédé de production de souches de virus de la poliomyélite modifiées fortement atténuées caractérisées en ce qu'elles présentent un taux de réversion réduit chez un hôte mammalien, ledit procédé comprenant:
a. l'isolement de séquences d'ARN du virus de la poliomyélite à partir de particules virales;
b. la préparation d'ADNc de virus de la poliomyélite à partir desdites séquences d'ARN de virus de la poliomyélite;
c. la mutagenèse dudit ADNc de virus de la poliomyélite dans la région correspondant à la séquence de bases 468-516 de la figure 4 afin de réduire l'auto-appariement de l'ADNc, de façon à ce que ladite mutagenèse réduise l'efficacité transcriptionnelle et traductionnelle des souches de virus de la poliomyélite qui sont produites à partir dudit ADNc du virus de la poliomyélite;
d. la transfection de cellules avec ledit ADNc du virus de la poliomyélite muté;
e. la culture desdites cellules hôtes; et
f. l'extraction des souches de virus de la poliomyélite à partir desdites cellules.

2. Procédé de production de souches de virus de la poliomyélite modifiées fortement atténuées caractérisées en ce qu'elles présentent un taux de réversion réduit chez un hôte mammalien, ledit procédé comprenant:
a. l'isolement de séquences d'ARN du virus de la poliomyélite à partir de particules virales;
b. la préparation d'ADNc du virus de la poliomyélite à partir desdites séquences d'ARN de virus de la poliomyélite;
c. la mutagenèse dudit ADNc du virus de la poliomyélite afin de réduire l'appariement des bases de l'ADNc avec l'ARN ribosomal dudit hôte mammalien, de façon à ce que ladite mutagenèse réduise l'appariement des bases dans les régions correspondant aux séquences de bases 449-460, 542-545, 547-551 et 555-565 dans la figure 4 et réduise par conséquent l'efficacité transcriptionnelle et traductionnelle des souches de virus de la poliomyélite qui sont produites à partir dudit ADNc du virus de la poliomyélite;
d. la transfection de cellules avec ledit ADNc du virus de la poliomyélite soumis à une mutagenèse;
e. la culture desdites cellules hôtes; et
f. l'extraction des souches de virus de la poliomyélite à partir desdites cellules.
